# FASCICULE DE BREVET EUROPEEN

(11) **EP 4 069 054 B1**
(45) Date de publication et mention de la délivrance du brevet: **25.03.2026**
(21) Numéro de dépôt: 20828033.9
(22) Date de dépôt: 02.12.2020
(51) Int. Cl.: A61B 1/00, A61B 1/005

(54) **MODULE D'ACTIONNEMENT MOTORISÉ D'UN INSTRUMENT ENDOSCOPIQUE**
MOTORISIERTES BETÄTIGUNGSMODUL ZUR BETÄTIGUNG EINES ENDOSKOPISCHEN INSTRUMENTS
MOTORIZED ACTUATION MODULE FOR ACTUATING AN ENDOSCOPIC INSTRUMENT

(30) Priorité: 03.12.2019 FR 1913678
(43) Date de publication de la demande: 12.10.2022
(73) Titulaire: Institut Hospitalo-Universitaire de Chirurgie Mini-Invasive Guidée par l'Image, 67091 Strasbourg (FR)
(72) Inventeur: SERRA-TORRENT, Albert, 08023 Barcelona (ES); LEGNER, Andras, 1220 Wien (AT); SKELTON, Eugene, Dublin, D06 K7 C0 (IE); WRIGHT, Anthony, Dunqarvan, X35 P042 (IE)
(74) Mandataire: IP Trust
(86) Numéro de dépôt international: PCT/FR2020/052253
(87) Numéro de publication internationale: WO 2021/111078

(56) Documents cités:
- EP-A1- 2 106 735
- EP-B1- 2 106 735
- WO-A1-2015/029041
- WO-A1-2017/025969

## Description

### Domaine de l'invention

La présente invention concerne le domaine de l'endoscopie flexible, plus particulièrement un système pour contrôler le mouvement de translation d'outils endoscopiques flexibles dans un endoscope flexible sortant. Un endoscope flexible comprend une poignée avec des boutons de commande qui permettent de déplacer l'extrémité flexible de la tige de l'endoscope dans différentes directions. L'utilisateur tient la poignée de la main gauche, dont les doigts peuvent actionner les différents boutons de règlage, pendant que la main droite guide la zone d'insertion de la tige flexible.

Initialement les endoscopes flexibles étaient surtout utilisés pour le diagnostic et notamment pour l'imagerie in vivo de corps creux. Mais les utilisations thérapeutiques des endoscopes flexibles se sont développées au cours des dernières décennies, mettant en œuvre des instruments de micro-chirurgie supplémentaires insérés via un canal de travail présent sur l'endoscope ou fixé à l'extérieur de l'endoscope. Ces instruments accessoires ont généralement au moins un mécanisme d'actionnement et/ou de déplacement et de positionnement de la tête distale active.

Lors d'une utilisation thérapeutique d'un endoscope flexible, l'utilisateur doit actionner et contrôler l'insertion, c'est-à-dire l'avancement et la rétraction, de l'instrument distal généralement avec la main droite. Au fur et à mesure que le mouvement se transmet le long de la tige endoscopique dont la zone d'introduction n'est plus guidée par la main de l'utilisateur, la position de la caméra se modifie et conduit à une perte de vision de la cible.

La coordination de ces actions perturbe souvent la continuité du flux de travail et entraîne un temps de procédure plus long. Pour résoudre ce problème, un deuxième opérateur / assistant est souvent nécessaire pour tenir la tige de l'endoscope, tandis que l'opérateur principal utilise la main libre pour contrôler manuellement l'outil flexible sur la poignée de l'endoscope flexible.

Cela nécessite des cycles d'apprentissage pour parvenir à une bonne communication et à une bonne coordination entre l'opérateur et l'assistant, sans éviter totalement les perturbations du travail, car il est souvent difficile de préciser l'angle, l'emplacement, le moment et une séquence de mouvements souhaités . Par conséquent, divers dispositifs de contrôle d'outils automatisés pour endoscopes flexibles ont été proposés pour résoudre ces difficultés .

### Etat de la technique

Pour faciliter la commande du déplacement de l'instrument sans recours à un assistant, on a proposé dans l'état de la technique des solutions où le déplacement est motorisé et où la poignée présente une commande du déplacement.

On connaît dans l'état de la technique la demande de brevet internationale WO2015/029041 décrivant une interface de paume pouvant être engagée par la paume d'une main, une retenue capable de se déformer élastiquement pour appliquer une force de retenue sur le dos de la main et une interface de doigt pouvant s'engager par un ou plusieurs doigts de ladite main.

La solution proposée par ce document comprend :
a) Une interface montée sur un support pivotant fixé à un boîtier de l'unité de commande, la première l'interface pouvant être engagée par la paume d'une main
b) un dispositif de retenue fixé de manière pivotante à la première interface et ayant un élément capable de se déformer élastiquement pour appliquer une force de retenue sur le dos de la main lorsque la paume est engagée avec la première interface; et
c) une seconde interface fixée de manière pivotante à la première interface et pouvant être engagée par un ou plusieurs doigts de la main.

On connaît aussi le brevet européen EP2106735 décrivant un endoscope configuré pour être porté avec une main, comprenant:
- une partie d'insertion s'étendant dans une direction longitudinale axiale, comprenant une partie d'extrémité distale et une partie d'extrémité proximale et configurée pour être insérée dans un corps; et
- une partie d'opération connectée de manière coaxiale à la partie d'extrémité proximale de la partie d'insertion, s'étendant dans la direction longitudinale axiale et configurée pour être tenue et actionnée par un opérateur, et
- dans lequel la partie d'insertion comprend une partie de courbure configurée pour être courbée.

La partie d'opération comprend:
- une première partie de préhension s'étendant dans la direction longitudinale axiale et configurée pour être saisie par un opérateur;
- un corps de partie d'opération de courbure agence sur un côté d'extrémité d'extrémité proximale dans la direction longitudinale axiale que la première partie de préhension et comprenant une partie d'extrémité proximale;
- étend dans le sens de la largeur et est configurée pour se déplacer le long de la direction longitudinale axiale par une rotation de la partie de support, dans lequel au moins l'un parmi un pouce et des doigts d'un opérateur peut être placé sur la partie de placement de doigts pour actionnement; et
- une unité radio connectée à la partie d'extrémité proximale du corps de partie d'opération de courbure et configurée pour réaliser une communication radio.

On connaît aussi la demande de brevet WO2017025969 décrivant

### Inconvénients de l'art antérieur

Un premier inconvénient des solutions de l'art antérieur concerne l'ergonomie des organes de commandes, et leur configuration qui n'est pas compatible avec les poignées d'endoscopes flexibles standards. Elles impliquent donc un temps d'apprentissage important pour le praticien. Certaines solutions nécessittent que les utilisateurs combinent le pouce et le majeur ou l'annulaire pour manipuler les molettes ce qui empêche l'actionnement simultané des vannes d'aspiration ou de lavage.

Un second inconvénient réside dans le fait que ces solutions nécessitent la reconception complète d'une poignée, et le remplacement des endoscopes existants par de nouveaux endoscopes.

La demande de brevet internationale WO2017025969 décrit une unité de commande pouvant être fixée à un endoscope flexible ayant une tige pouvant être déformée via deux boutons rotatifs. L'unité de commande comprend une interface utilisateur comprenant une première interface montée sur un support pivotant fixé à un boîtier de l'unité de commande, la première interface pouvant être engagée par la paume d'une main. L'unité de commande comprend en outre une unité d'entraînement pouvant fonctionner via l'interface utilisateur, l'unité d'entraînement comprenant un premier mécanisme d'entraînement pour engager les deux boutons rotatifs permettant ainsi à un utilisateur de contrôler la déflexion de l'arbre de l'endoscope via la première interface.

### Solution apportée par l'invention

La présente invention vise à éviter les inconvénients de l'art antérieur sous deux formes de réalisation, soit sous forme d'un module séparé formant un accessoire permettant d'upgrader une poignée d'endoscope commercial, soit sous forme de poignée d'endoscope présentant nativement un tel module de commande. Dans les deux modes de réalisation, l'invention est constituée par une platine apte à être fixée sur la zone de préhension cubito-palmaire d'une poignée d'un endoscope, ladite platine présentant une surface d'appui cubito-palmaire prolongée par un talon s'étendant dans une direction formant un angle de 90° ± 25° par rapport à la perpendiculaire au plan de ladite surface d'appui cubito-palmaire, ledit talon comportant un capteur électro-mécanique délivrant un signal de commande du déplacement d'un instrument endoscopique.

On entend par « platine » au sens du présent brevet une lame fine, d'une épaisseur inférieure à 2 millimètres, plane ou déformée pour permettre à la face arrière d'épouser la surface de la zone de préhension cubito-palmaire d'une poignée d'un endoscope.

On entend par « zone de préhension cubito-palmaire d'une poignée d'un endoscope » la surface latérale semi-tubulaire de la poignée s'étendant entre les deux extrémités frontales transversales de la poignée. La paume de la main entoure la poignée de forme générale tubulaire, le pouce s'étendant d'un côté et les doigts (index, majeur, annulaire et éventuellement auriculaire) venant épouser de l'autre côté la zone de préhension cubito-palmaire.

L'invention concerne plus particulièrement un module d'actionnement motorisé d'un instrument endoscopique présentant les caractéristiques énoncés par la revendication 1.

Le module est formé par une platine apte à être fixée sur la zone de préhension cubito-palmaire d'une poignée d'un endoscope, ladite platine présentant une surface d'appui cubito-palmaire prolongée par un talon s'étendant dans une direction formant un angle de 90° ± 25° par rapport à la perpendiculaire au plan de ladite surface d'appui cubito-palmaire , ledit talon comportant un capteur électro-mécanique délivrant un signal de commande du déplacement d'un instrument endoscopique.

Avantageusement, ladite surface de préhension cubito-palmaire médiane est prolongée du coté opposée par un bloc d'entraînement motorisé, comportant un mécanisme motorisé d'un élément filiforme de liaison avec ledit instrument dont l'extrémité inférieure débouche dans ladite tige flexible pour assurer une liaison avec ledit instrument distal, ladite platine présentant en outre des moyens de liaison avec la poignée d'un endoscope flexible.

Avantageusement, lesdits moyens de liaison avec la poignée d'un endoscope flexible sont constitués par un embout apte à être insérré dans le canal opérateur d'une tige endoscopique.

Selon une variante préférée, la platine comporte un bloc d'entraînement motorisé comportant un moteur placé en-dessous de l'embout avec l'entrée du canal opérateur d'un endoscope associé.

Avantageusement, ledit talon est formé par une protubérance d'épaisseur inférieure à 3 millimètres présentant à son extrémité un capteur dont la surface d'actionnement est définie par une génératrice formant un angle de angle de 90° ± 20° avec l'axe longitudinal de ladite poignée.

Selon un mode de réalisation particulier, le bloc d'entraînement, comportant un mécanisme motorisé d'un élément filiforme de liaison avec ledit instrument dont l'extrémité inférieure débouche dans ladite tige flexible de l'endoscope pour assurer une liaison avec ledit instrument distal.

Selon une variante, ledit bloc d'entrainement présente un bouton d'actionnement latéral commandant l'arrêt d'urgence du déplacement dudit instrument.

L'invention concerne aussi un endoscope souple comportant une poignée présentant des boutons de commande de l'aspiration et du lavage située au-dessus d'une zone de préhension cubito-palmaire des doigts de l'utilisateur, ladite poignée étant prolongée par une tige flexible présentant un canal opérateur pour le passage d'un instrument dont le déplacement est commandé par une interface électrique caractérisé en ce que ladite interface présente une surface d'appui cubito-palmaire prolongée par un talon s'étendant dans une direction formant un angle de 90° ± 25° par rapport à la perpendiculaire au plan de ladite surface d'appui cubito-palmaire , ledit talon comportant un capteur électro-mécanique délivrant un signal de commande du déplacement d'un instrument endoscopique.

Avantageusement, ledit talon est formé par une protubérance d'épaisseur inférieure à 3 millimètres présentant à son extrémité un capteur dont la surface d'actionnement est définie par une génératrice formant un angle de angle de 90° ± 20° avec l'axe longitudinal de ladite poignée.

Avantageusement, ledit capteur est un capteur rotatif actionné par une molette dont l'axe forme un angle compris entre 0° et ±70° par rapport à l'axe longitudinal parallèle à l'axe longitudinal de ladite poignée.

Avantageusement, la platine comprend en outre dans la partie inférieure de ladite zone de préhension cubito-palmaire, un bloc d'entraînement comportant un mécanisme motorisé d'un élément filiforme de liaison avec ledit instrument dont l'extrémité inférieure débouche dans ladite tige flexible de l'endoscope pour assurer une liaison avec ledit instrument distal.

De préférence ledit bloc d'entrainement forme une protubérance par rapport à la surface de la partie inférieure de la zone de préhension cubito-palmaire, à l'opposé, par rapport à ladite zone de préhension cubito-palmaire, audit prolongement consituant la commande d'actionnement.

Avantageusement, ladite interface de commande (100) comporte en outre un sélecteur de longueur pour le déplacement de l'instrument d'une longeur prédéterminée.

De préférence, ladite interface de commande comporte en outre un moyen d'accouplement d'un périphérique dudit instrument.

Selon une variante l'endoscope comporte un module d'actionnement motorisé regroupant sous forme d'un sous-ensemble détachable de la poignée ladite interface et ledit bloc d'entraînement.

Selon une autre variante, ledit capteur présentant une surface d'interaction opposée à ladite zone de préhension cubito-palmaire inférieure à 50 mm².

### Description détaillée d'un exemple non limitatif de réalisation

L'invention est décrite ci-après, à titre d'exemple non limitatif, par référence aux dessins annexés dans lesquels :
[Fig. 1] la figure 1 est une vue en perspective partielle éclatée montrant une poignée d'endoscope selon l'état de la technique
[Fig. 2] la figure 2 est une vue de 3/4 face d'une poignée d'endoscope selon l'invention
[Fig. 3] la figure 3 représente une vue de 3/4 face d'une poignée d'endoscope selon l'invention

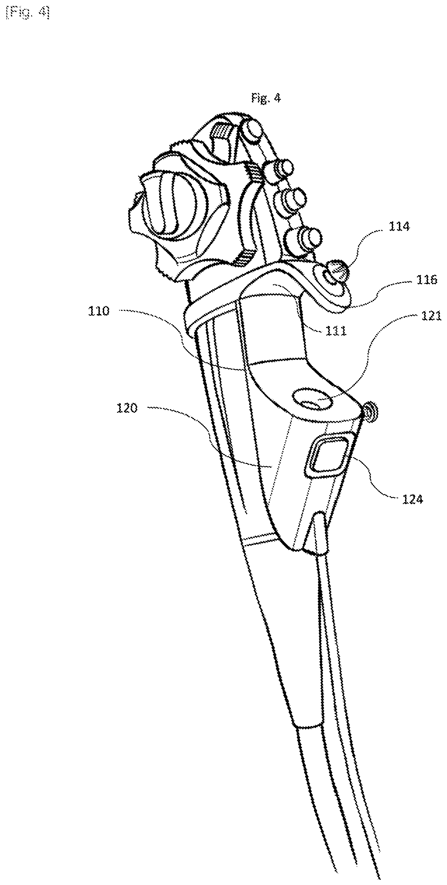

[Fig. 4] la figure 4 est une vue de de 3/4 face d'une deuxième variante de poignée d'endoscope selon l'invention
[Fig. 5] la figure 5 représente une vue de 3/4 face d'une première variante d'un module de commande selon l'invention
[Fig. 6] la figure 6 représente une vue de 3/4 face d'une deuxième variante d'un module de commande selon l'invention
[Fig. 7] la figure 7 est une vue de 3/4 face d'une troisième variante de poignée d'endoscope selon l'invention
[Fig. 8] la figure 8 est une vue de 3/4 face d'une troisième variante de poignée d'endoscope selon l'invention
[Fig. 9] la figure 9 est une vue de 3/4 face d'une deuxième variante d'un module selon l'invention
[Fig. 10] la figure 10 est une vue du mécanisme intérieur de cette deuxième variante d'un module selon l'invention
[Fig. 11] la figure 11 est une vue en coupe longitudinale de cette deuxième variante d'un module selon l'invention
[Fig. 12] la figure 12 est une vue de dessous de cette deuxième variante d'un module selon l'invention
[Fig. 13] la figure 13 est une vue en perspective de l'embout de liaison avec le canal opératoire de l'endoscope
[Fig. 14] la figure 14 est une vue en perspective d'une première configuration d'une platine selon l'invention
[Fig. 15] la figure 15 est une vue en perspective d'une deuxième configuration d'une platine selon l'invention
[Fig. 16] la figure 16 est une vue en perspective d'une troisième configuration d'une platine selon l'invention
[Fig. 17] la figure 17 est une vue en perspective d'une quatrième configuration d'une platine selon l'invention
[Fig. 18] la figure 18 est une vue en perspective d'une cinquième configuration d'une platine selon l'invention
[Fig. 19] la figure 19 est une vue en perspective d'une sixième configuration d'une platine selon l'invention.

### Contexte général de l'invention : les poignées d'endoscope « standards » de l'état de la technique.

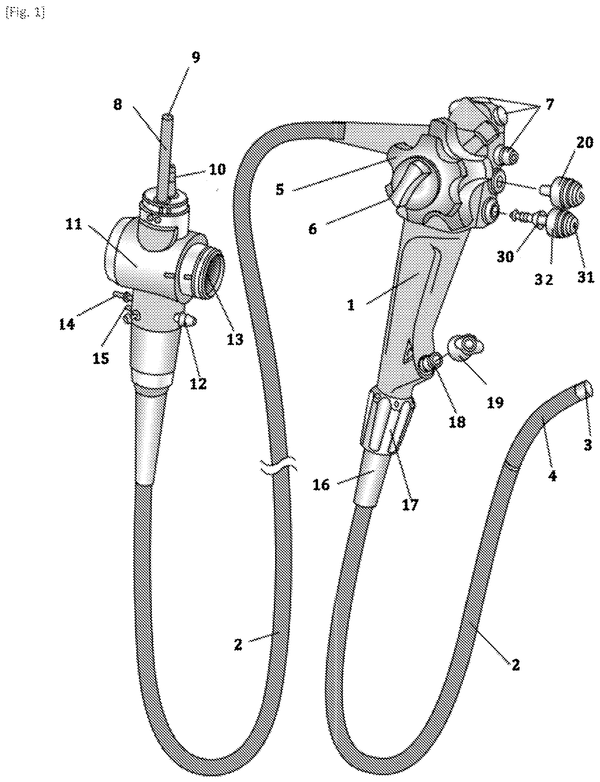

La figure 1 représente un exemple d'endoscope connu. Il comporte une poignée (1) à partir de laquelle s'étend un tube souple (2) se terminant par une extrémité distale ou opérationnelle (3) qui est introduite dans la cavité à explorer et dont l'orientation peut être commandée par un manchon béquillable (4). Ce manchon est commandé par des fils qui sont tirés de manière contrôlée par suite de l'actionnement manuel de molettes ou de boutons (5, 6, 7) prévus sur la poignée (1) d'endoscope. La section de commande des manettes mobiles permet au médecin de commander toutes les fonctions de l'endoscope. Les manettes (5, 6, 7) de béquillage (orientation dans l'espace) dirigent les câbles de béquillage et contrôlent la section béquillable à l'extrémité distale du tube d'insertion, permettant ainsi une orientation bidimensionnelle. Des mécanismes de blocage (freins) permettent de fixer la section béquillable dans la position voulue.

La lumière est transmise par une fibre optique (8) dont l'extrémité proximale (9) est accouplée à un connecteur optique. Le bloc de raccordement (11) comprend en outre une prise d'air (10).

Un raccord pneumatique (12) est destiné à être raccordé à une pompe à vide ou une pompe à air réversible pour commander l'insufflation ou l'aspiration à l'extrémité distale (3). Le bloc technique (11) comporte également un connecteur électrique (13) et un support de raccordement (14) d'un cordon de sécurité et une alimentation en eau (15).

La poignée (1) est raccordé sur le tube (2) par une gaine (16) prolongée par un manchon (17). La poignée comporte une ouverture du canal opérateur (18) équipée d'une valve jetable (19).

La poignée comporte un piston (20) commandant le canal de lavage et un piston (30) de commande de l'aspiration ou d'insufflation par l'intermédiaire de deux tubes s'étendant entre la poignée (1) et l'extrémité distale (3), l'un pour amener de l'air et de l'eau à ladite extrémité distale, l'autre pour effectuer une biopsie ou une aspiration. L'application d'une aspiration est réglée au moyen d'un piston (30) prévue sur la poignée. Le piston est réuni à un conduit voisin par des liaisons soudées. Les pistons (20, 30) sont accouplés sur le corps de la poignée par des embases annulaires (32).

Le piston (30) relie le canal d'aspiration au canal opérateur dans le tube d'insertion. En appuyant sur le bouton (31) du piston, on peut effectuer une aspiration du canal opérateur. Le piston air/eau (20) est semblable au piston et au piston d'aspiration (30), sauf qu'un piston avec bouton à deux voies est utilisée dans un dispositif à double canal qui permet de véhiculer l'air ou l'eau jusqu'à la lentille à l'extrémité distale, pour le lavage ou l'insufflation d'air afin d'améliorer la vision. Les deux pistons (20, 30) peuvent être détachées pour le remplacement, lorsqu'elles sont à usage , ou sinon pour le nettoyage.

### Description détaillée de l'interface de commande

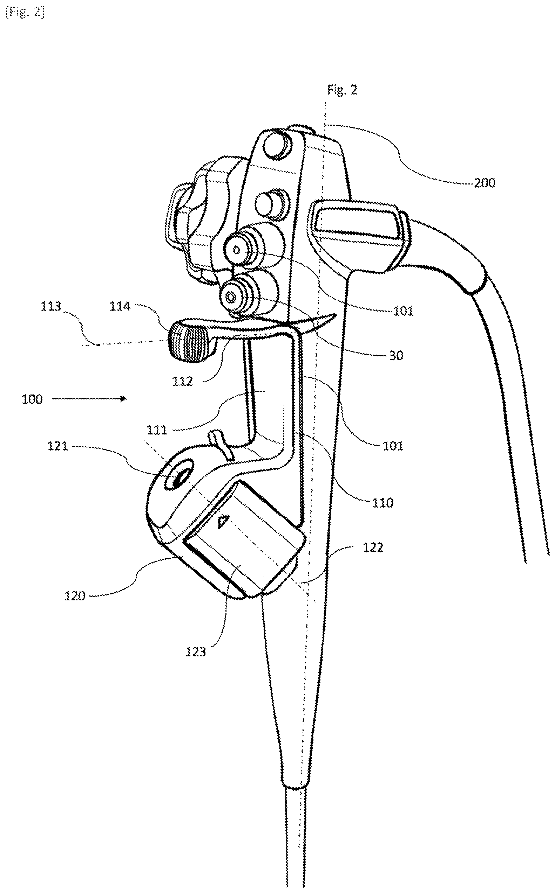

La poignée selon l'invention représentée par la figure 2 et suivantes, se distingue des poignées « standards » par une fonction additionnelle de commande motorisée d'un instrument endoscopique introduit dans le tube endoscopique (2) pour effectuer à l'extrémité distale une intervention telle qu'un prélèvement, une électro-coagulation, une suture, un incision, etc... Cette commande motorisée est destinée à faire avancer ou reculer l'instrument endoscopique dans le tube endoscopique (2), sans qu'un opérateur n'ait besoin de pousser ou tirer l'instrument. Cette commande motorisée ne concerne pas l'éventuel actionnement de l'extrémité de l'instrument endoscopique, par exemple l'ouverture ou la fermeture d'une pince prévue à l'extrémité distale de l'instrument endoscopique.

Le but est de permettre à l'opérateur tenant la poignée (1) d'une main de continuer à exercer les commandes usuelles, mais aussi de commander sans intervention manuelle le déplacement de l'instrument endoscopique jusqu'à sa position de travail, sans nécessiter le recours de l'autre main, généralement utilisée pour guider le tube endoscopique (2) dans la zone d'introduction, et sans recourir à l'assistance d'un deuxième opérateur, nécessitant une coordination complexe. Le déplacement de l'instrument consiste à progresser dans le tube endoscopique selon une trajectoire correspondant à la médiane du tube, en avant et en arrière, pour permettre à la partie active de l'instrument d'atteindre la zone d'intervention. Il s'agit d'un déplacement par rapport au tube endoscopique (2).

Pour cela, la poignée (1) est équipée d'une interface de commande (100) venant se positionner sur la poignée (2) au niveau de la zone de préhension cubito-palmaire (101) juste en-dessous du piston (20) commandant le canal de lavage et du piston (30) de commande de l'aspiration ou d'insufflation. Cette zone (101) est généralement plate pour permettre l'appui de la palme d'au moins deux ou trois doigts, de l'index, du majeur, de l'annulaire et de l'auriculaire.

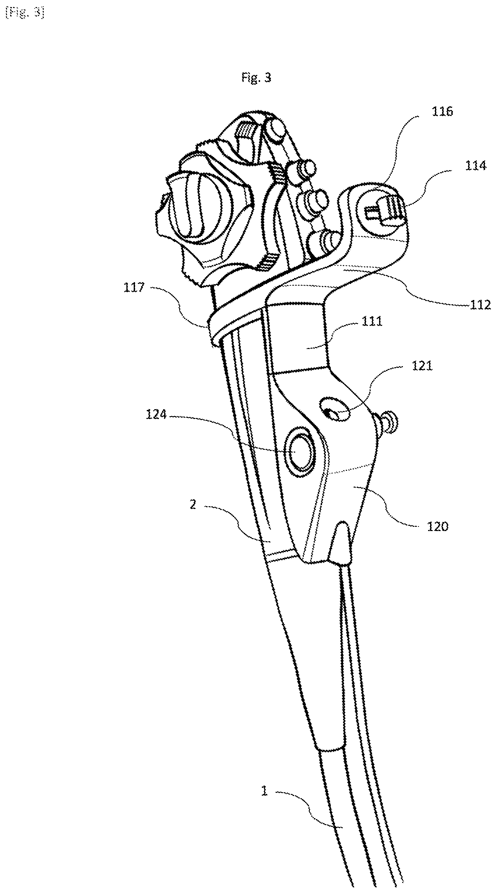

Le module illustré par les figures 2 et 3 est constitué par une platine (110) de faible épaisseur, environ 1 millimètre, présentant une partie médiane (111) d'appui cubito-palmaire venant se superposer à la zone de préhension cubito-palmaire (101) de la poignée (2), s'étendant latéralement sur la poignée. Cette partie médiane (111) est prolongée d'un coté par un prolongement (112) s'étendant dans un plan sensiblement transversal (113) . On entend par «plan sensiblement transversal » au sens du présent brevet une surface s'étendant dans une direction comprise entre +45° et -45° par rapport à l'axe longitudinal (200) de la poignée (2).

Ce prolongement (112) présente une largeur décroissante, depuis une largeur initiale correspondant à la largeur de la zone médiane (111), jusqu'à une extrémité de moindre largeur supportant le capteur muni d'une surface d'actionnement (114). Dans l'exemple dérit le capteur est un capteur électro-mécanique rotatif avec une surface d'actionnement (114) formé par une molette crantée en forme de zone sphérique permettant l'actionnement avec la palme d'un doigt, par exemple le majeur ou l'annulaire, laissant l'index disponible pour les autres commandes habituelles de la poignée.

L'autre extrémité de la platine (100) est formée par un bloc motorisé (120) dont l'axe longitudinal (122) est incliné par rapport à l'axe longitudinal (200) de la poignée (2), avec un angle compris entre ±30° (incliné vers le haut ou le bas) et 90° (orientation perpendiculaire à l'axe longitudinal (200) de la poignée (2)).

Ce bloc motorisé (120) présente un canal médian s'ouvrant de chaque coté du bloc (120) par un orifice (121) pour le passage de l'élément de translation de l'instrument endoscopique, pour assurer son déplacement à l'intérieur du tube endoscopique (1) et son positionnement distal pour une intervention avec cet instrument. A cet effet, le bloc (120) comprend un moteur électrique ou un actionneur électromagnétique commandé par le capteur (114) par l'intermédiaire d'un circuit électronique de pilotage. Il est alimenté par une batterie électrique rechargeable (123) engagée dans un connecteur latéral. Sur la paroi opposée, le bloc (120) présente un bouton d'arrêt d'urgence (124).

Dans l'exemple de réalisation illustré par la figure 2, le prolongement (112) est recourbé vers le haut à son extrémité (116), qui porte le capteur (114). Par ailleurs la platine est munie d'un collier (117) pour la fixation autour d'une poignée (2) standard.

Dans la variante illustrée par la figure 3, le bouton d'arrêt d'urgence (124) est disposé sur la face avant du bloc motorisé (120), et la platine (110) présente un prolongement (111) s'étendant transversalement, avec une extrémité (116) inclinée vers le haut pour supporter un capteur (114) actionnable par un bouton hémisphérique.

### Réalisation sous forme d'un module-accessoire

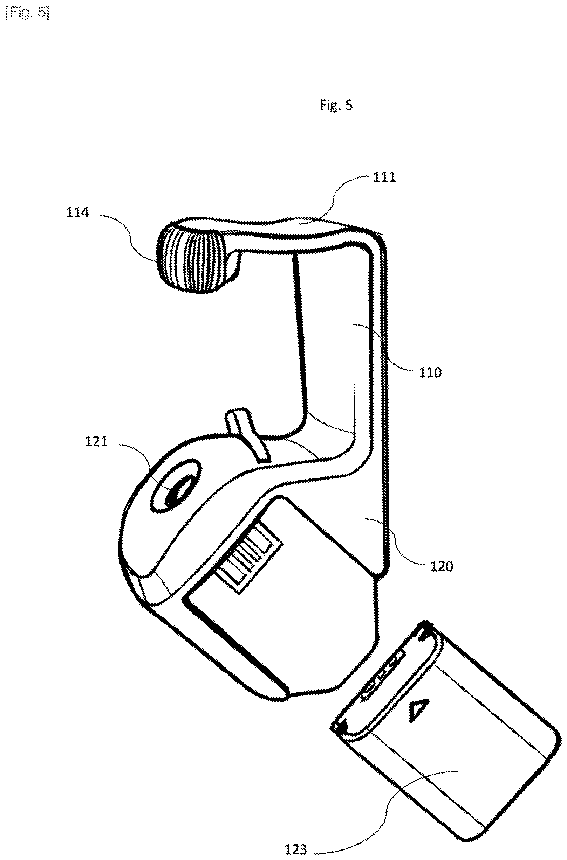

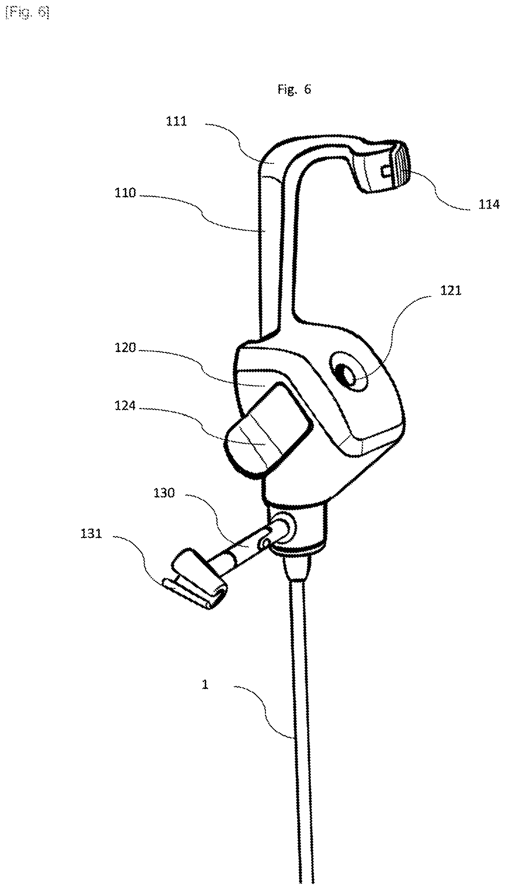

L'interface peut être intégrée à une poignée (2) ou être réalisée sous forme d'un accessoire qui peut être monté et fixé sur une poignée (2) pré-existante. Les figures 5 et 6 illustrent des vues d'un tel accessoire, présentant les mêmes caractéristiques techniques que celles précédemment décrite.

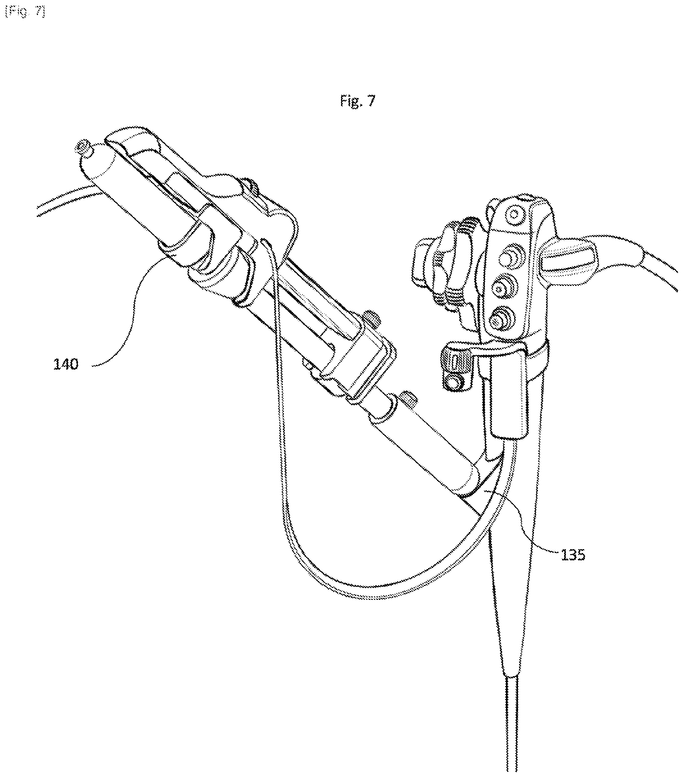

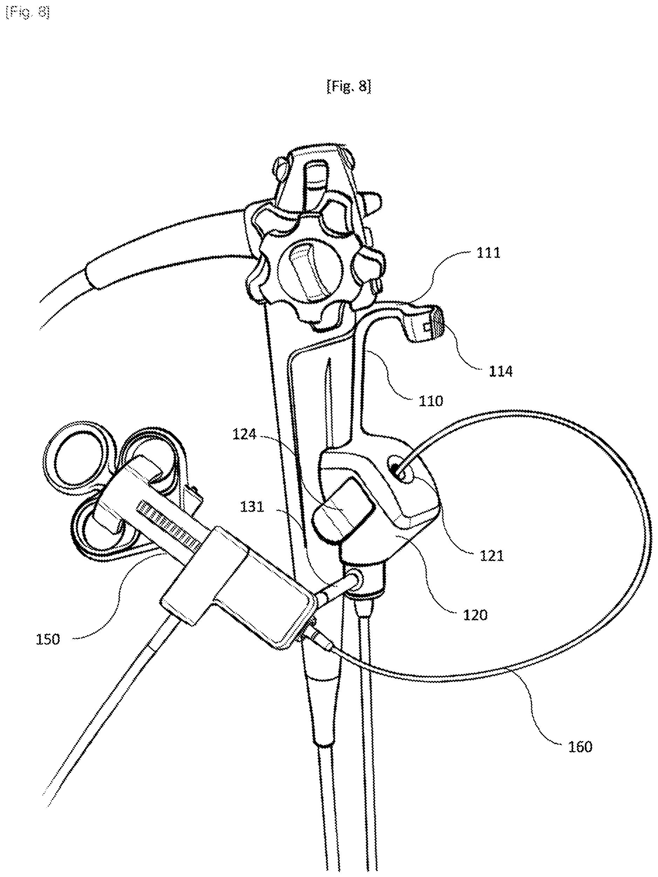

Le bloc motorisé (120) est prolongé latéralement par adaptateur pour recevoir un périphérique à l'instrument endoscopique, par exemple un équipement automatisé de ponction (140) comme représenté en figure 7 ou un mécanisme (150) de commande de ciseaux ou une aiguille de biopsie comme représenté en figure 8.

Cet adapteur est constitué par un bras articulé (130) munie d'un support d'accessoire (131) ou par un connecteur mécanique (135).

### Variante de réalisation d'un module-accessoire

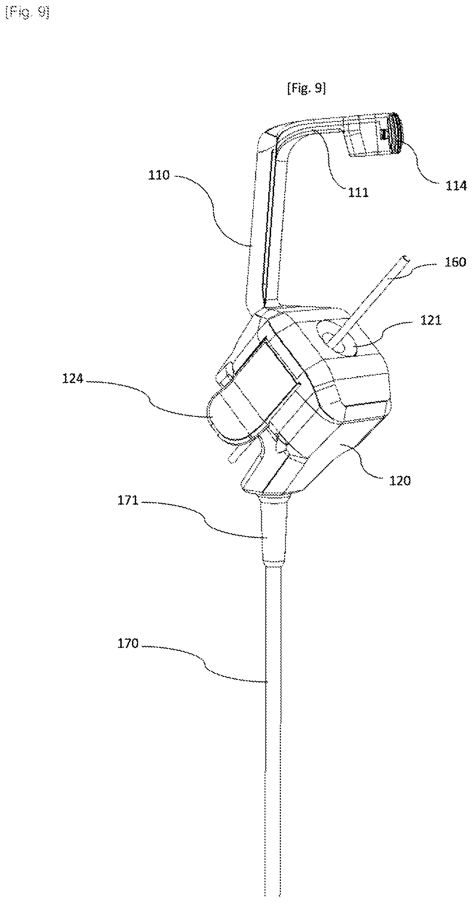

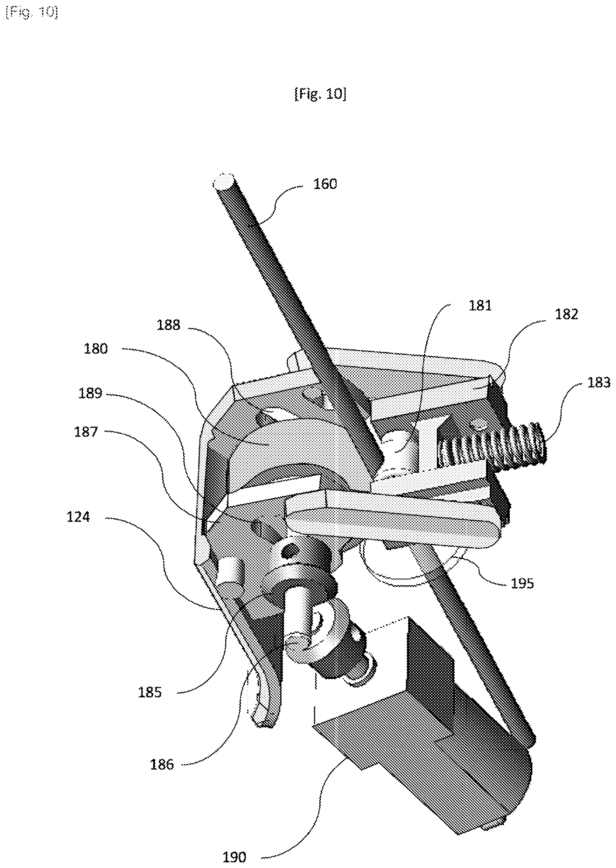

Les figures 9 et 10 représente une variante de réalisation d'un module autonome, constituant un accessoire destiné à équiper une poignée endoscopique pré-existante. Les caractéristiques techniques précédement décrites sont présentes sur cette variante.

Le bloc motorisé (120) intègre un mécanisme d'entraînement de l'élément filiforme (160) par un galet d'entraînement (180) venant en contact avec la surface de cet élément filiforme (160), un galet-presseur (181) venant en contact diamètralement opposée pour assurer une bonne adhérance. Ce galet presseur (181) tourne librement autour d'un axe supporté par un chariot mobile (182) repoussé en direction du galet (180) par un ressort (183) orienté perpendiculairement à l'axe d'introduction de l'élément filiforme (160). Il est alimenté par un fil électrique (170) protégé par un manchon (171).

Le galet d'entraînement (180) est actionné par un moteur (190) par l'intermédiaire d'un engrenage (185) de renvoi d'angle. Ce moteur (180) est commandé par l'intermédiaire de la commande utilisateur (114).

Le moteur (190) est disposé selon une direction sensiblement longitudinale, par rapport à l'axe de défilement de l'élément filiforme (160), avec un angle compris entre 0° et ± 30° par rapport à cet axe pour optimiser l'encombrement et la répartition des masses.

Le moteur (190) est positionné dans le bloc motorisé (120) en-dessous de l'interface (195) venant se placer sur l'entrée du canal opérateur de l'endoscope, afin de favoriser un bon équilibrage de la poignée et éviter de remonter le centre de gravité de la poignée équipée de ce module.

L'axe (186) est guidé par deux lumières arquées (188, 189) formée sur un batin (187) solidaire du bouton d'actionnement (124).

En position intermédiaire du bouton d'actionnement (124), le galet d'entraînement (180) assure le déplacement de l'élément filiforme (160) qui est appuyé avec une force contrôlée contre lui par le galet libre (181).

Lorsqu'un repousse le bouton d'actionnement (124) en direction de l'axe de défilement de l'élément filiforme (160), en direction de la poignée lorsque le module est monté sur la poignée, on provoque le désaccouplement des engrenages du levier d'angle (185) conjointement au pressage du galet d'entraînement (180) contre le galet libre (181), ce qui bloque le défilement de l'élément filiforme (160).

Lorsqu'on déplace au contraire le bouton d'actionnement (124) en direction opposée, en agissant sur sa partie recourbée par un doigt glissé entre la poignée et la surface intérieure du bouton d'actionnement (124), on écarte le galet d'entraînement (180) du le galet libre (181) et on libère ainsi l'élément filiforme (160) qui peut alors être déplacé manuellement.

L'exploitation de ce bouton d'actionnement (124) est très intuitive. Il commande l'ouverture ou la fermeture instantanée du mécanisme d'engagement de l'outil :
- engagement / désengagement instantané de l'outil déplacé par l'élément filiforme (160)
- sécurité par arrêt immédiat pour avance / retrait de l'outil déplacé par l'élément filiforme (160)
- commutation instantanée entre fonctionnement manuel et automatisation

Le fonctionnement manuel de l'outil permet de revenir à une utilisation habituelle de l'endoscope, sans avoir à retirer le module motorisé.

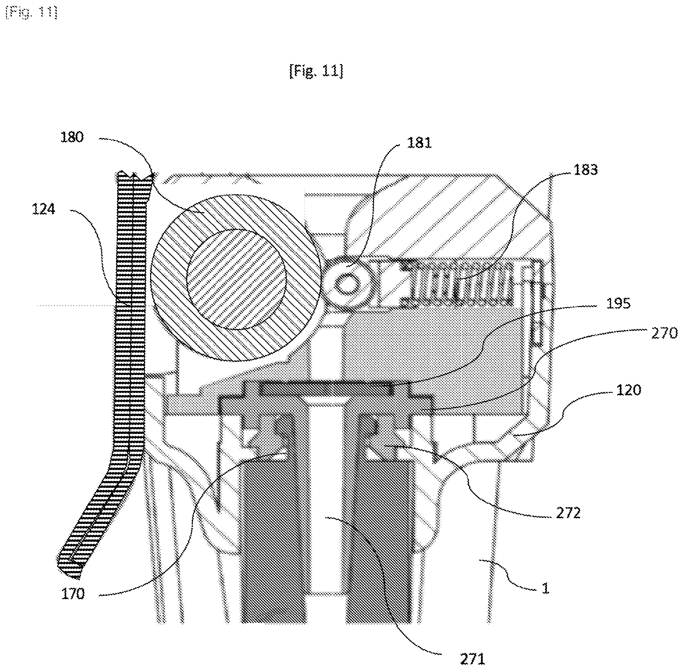

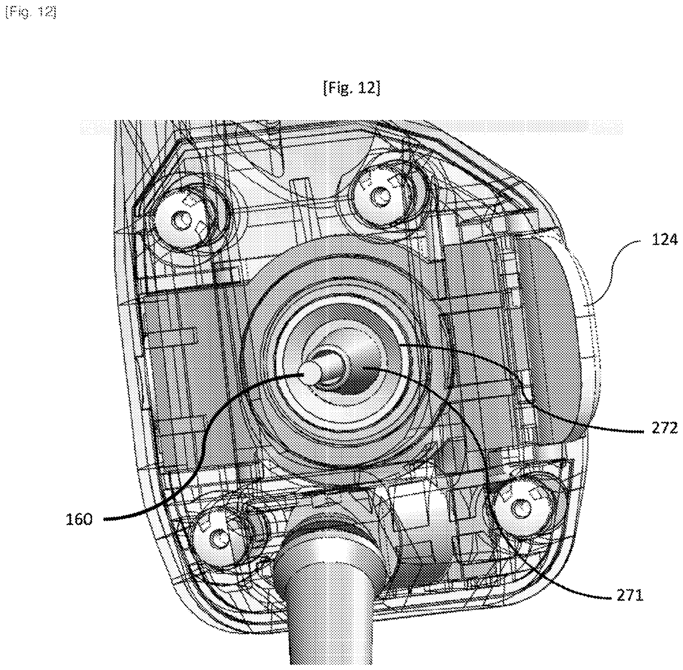

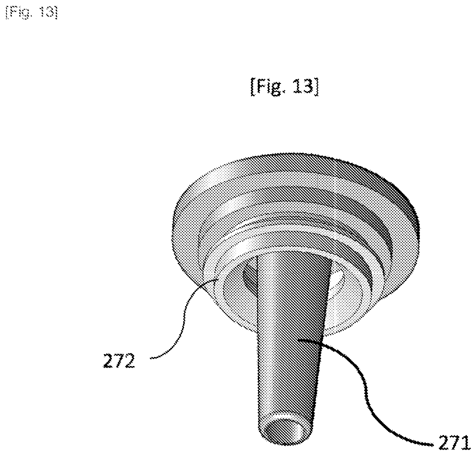

### Fixation de l'instrument sur le canal opérateur de l'endoscope (figure 11 à 13)

La fixation du module sur la poignée ne nécessite pas d'élément de fixation tel qu'un collier ou un clip, mais est réalisée par une pièce tronconique représentée en figure (13), présentant une collerette (270) assurant l'étanchéité entre le canal opérateur et le module motorisé, prolongé par un embout tronconique (271). L'introduction dans le canal opérateur d'un endoscope « standard » de l'embout longitudinal (271) prolongeant l'extrémité distale du bloc motorisée (120) assure à la fois le guidage de l'élément filiforme (160) et le calage du module par rapport à la poignée endoscopique. La couronne (271) de forme générale tubulaire ou tronconique présente un joint (272) qui assure l'étanchéité de la gaine (170) par rapport au guide endoscopique. Ce joint (272) comporte un bourrelet venant s'engager par déformation et coincement autour de l'extrémité du canal opérateur (comportant elle aussi un bourrelet).

L'embout tronconique (271) présente à son extrémité proximale un épanouissement discal (270) venant s'engager dans une zone de réception complémentaire prévue à la face frontale du bloc motorisé (120). L'extrémité proximale de l'embout (271) présente un espace circulaire dans lequel vient s'engager un joint (195). Cette solution permet de séparer physiquement le canal opérateur du guide (1), du moteur et du mécanisme d'entraînement constituant partie supérieure du bloc motorisé (120). Un ou plusieurs joints de fluide scellent le fluide dans le passage de la tige (1).

### Applications

Les applications de l'invention sont multiples, elles concernent notamment le prélèvement écho-endoscopiquement guidé à l'aiguille fine (EUS-FNA), avec étude cytologique et histologique. L'échographie endoscopique (EUS) est devenu un protocole essentiel pour la la détermination du stade d'avancement du cancer dans le système gastro-intestinal, en particulier lorsqu'elle est combinée à une aspiration à l'aiguille fine (FNA) ou à une biopsie à l'aiguille fine (FNB) des tissus corporels. La biospsie FNA est réalisée à l'aide d'une aiguille dédiée appelée aiguille FNA. Au cours de la procédure, un imageur EUS est mis en contact avec une paroi corporelle derrière laquelle se trouve le site de biopsie, puis une aiguille FNA est avancée dans le canal de travail de l'endoscope EUS. L'aiguille est avancée à travers la paroi corporelle vers le site à biopsier, généralement une lésion suspecte, et une pression négative est appliquée à l'extrémité interne de l'aiguille pour récupérer des échantillons de tissu. L'aiguille est ensuite extraite du champ EUS et l'échantillon de tissu est récupéré et analysé.

Les aiguilles FNA disponibles sur le marché sont assez similaires: elles sont destinées à être engagées à l'extrémité proximale du canal de travail (1), généralement via le verrouillage Luer et comprennent une aiguille creuse logée dans une gaine. Le manche de l'extrémité proximale de l'aiguille FNA comprend:
- un ajusteur de gaine utilisé pour mettre l'extrémité de la gaine en contact avec la paroi corporelle à traverser (avec l'aiguille non exposée). Une fois que la position correcte de la gaine est atteinte, le dispositif de réglage de la gaine peut être sécurisé par rapport au corps de la poignée.
- un curseur d'aiguille permettant de glisser et d'exposer l'aiguille au-delà de l'extrémité distale de la gaine pour atteindre le site de biopsie
- une bague de sécurité qui peut être fixée au corps de la poignée pour limiter la course longitudinale du curseur d'aiguille de sorte que plusieurs insertions d'aiguille puissent être facilement effectuées par l'utilisateur.
- un orifice à l'extrémité proximale du manche pour appliquer une pression négative sur l'alésage intérieur creux de l'aiguille.

Une biopsie par FNA nécessite généralement que le médecin passe l'aiguille de biopsie plusieurs fois manuellement, dans les deux sens, à différents endroits dans la lésion cible. L'action de passer l'aiguille de biopsie n'est pas normalisée, car la profondeur de pénétration et la vitesse de passage sont contrôlées par les médecins. Habituellement, un long passage reçoit généralement plus d'échantillon que des passes plus courtes; et une passe lente reçoit généralement plus d'échantillon que des passes plus rapides. Un échantillonnage non normalisé pourrait entraîner un rendement sous-optimal ou un échantillonnage par biopsie non reproductible. La méthode conventionnelle produit également une mauvaise estimation du rendement de l'échantillon, ce qui peut entraîner un échantillonnage insuffisant et un échec du diagnostic. Cela fait perdre du temps, retarde probablement le traitement des patients et est associé à des coûts médicaux supplémentaires qui pourraient être évités grâce à une méthode d'échantillonnage plus standardisée. Un problème technique similaire a été mentionné dans le brevet DE10128336, une méthode a été proposée. Cette métode de prélèvement d'échantillons de cellules par biopsie par aspiration à l'aiguille fine (FNA) consiste à introduire une aiguille fine d'un appareil de prélèvement dans le tissu, cette aiguille fine étant soumise à une dépression et elle est mise en mouvement par rapport au tissu, puis la dépression est stoppée et l'aiguille fine est retirée du tissu.

La présente invention propose un dispositif avec une interface de contrôle d'outil qui améliore le contrôle de l'échantillonnage d'aiguille de biopsie, normalisant l'échantillonnage de tissu qui permet une meilleure reproductibilité, la planification et l'estimation d'échantillons de tissu.

Avec les poignées de l'art antérieur, l'actionnement du curseur de l'aiguille impose à l'endoscopiste de laisser sa main droite sur l'axe de la lunette de visée endoscopique, ce qui se traduit par une perte de contrôle de l'endoscope et une diminution de la précision. C'est une limitation importante car atteindre différents emplacements dans la lésion est critique pour le rendement diagnostique. Un système permettant à l'utilisateur de faire fonctionner une aiguille FNA tout en maintenant une main sur la poignée et l'autre sur la tige de l'endoscope est donc souhaitable.

L'interface de commande selon l'invention peut avantageusement être utilisée pour contrôler une aiguille FNA, l'utilisateur gardant sa main droite sur la tige de l'endoscope. Le mouvement de l'arbre et de l'aiguille peut être contrôlé en actionnant 1 interface de contrôle.

Dans un mode de réalisation préféré, l'insertion de l'aiguille est contrôlée par la main droite en utilisant l'interface de contrôle. une commande d'entraînement à une dimension permet un fonctionnement lent et précis de l'aiguille. Une fois l'aiguille insérée à la profondeur souhaitée, un deuxième contrôle d'échantillonnage booléen (activé / désactivé) peut être utilisé pour répéter le mouvement: une fois que vous appuyez sur le contrôle d'échantillonnage, l'aiguille est rétractée et réinsérée à la même distance. Cette fonctionnalité permet à l'utilisateur de faire plusieurs passes avec une profondeur similaire tout en faisant varier la position de la portée (technique de ventilation) pour atteindre plusieurs sites au sein de la lésion. L'aiguille peut ensuite être retirée à l'aide du contrôleur d'entraînement et elle peut être retirée de l'endoscope.

Selon une variante, l'interface de commande (100) comporte en outre un sélecteur de longueur pour permettre à l'utilisateur de définir directement la profondeur de pénétration de l'aiguille. Lorsque l'utilisateur appuie sur la commande d'échantillonnage, l'aiguille est insérée jusqu'à la profondeur définie par le sélecteur de longueur et rentrée dans une position par défaut.

Dans un mode de réalisation, l'interface de contrôle (100) est utilisée conjointement avec une aiguille FNA intégrant au moins un actionneur linéaire adapté pour translater l'aiguille. L'actionneur est connecté à l'interface de commande physiquement ou sans fil. L'aiguille FNA est également connectée à une source d'alimentation, soit via une connexion physique avec l'interface de contrôle, soit en intégrant une batterie.

Dans un autre mode de réalisation, l'interface de contrôle est connectée à un «dispositif EUS» adapté pour être monté sur une aiguille FNA disponible et connectée à l'interface de contrôle.

### Résumé de l'application « biopsie »

1) Un endoscope selon l'invention, ou un endoscope standard complété par un module selon l'invention comprend deux parties destinées au fonctionnement de l'instrument endoscopique: une interface de contrôle utilisateur et une unité d'entraînement.
2) l'interface de contrôle utilisateur est montée sur la poignée (2) de l'endoscope et juste en dessous des boutons d'aspiration et d'irrigation (20, 30). La commande utilisateur (114) comporte un premier bouton de commande pour appliquer un ordre de déplacement relativement lent; et optionnellement un bouton d'échantillonnage qui avance et rétracte continuellement l'aiguille de biopsie pour un nombre fixe de passes à la longueur prédéfinie, définie par le sélecteur de longueur.
3) Avant de fixer l'unité d'entraînement sur le viseur, la bague de sécurité de l'aiguille doit être bien serrée, idéalement à la longueur maximale. Cela permet à l'aiguille de parcourir la distance maximale et d'avoir le point de poids central plus près de la poignée de la lunette.
4) L'unité d'entraînement est constituée d'un actionneur linéaire composé de 2 parties, une partie non coulissante et une partie coulissante. Chaque partie contenant un moyen de fixation qui est monté sur le dispositif d'aiguille à biopsie. Le moyen de fixation sur la partie non coulissante est fixé sur la bague de sécurité du dispositif à aiguille. Le moyen de fixation sur la partie coulissante est fixé sur la poignée coulissante du dispositif de biopsie.
5) L'entraînement comprend également un mécanisme de sécurité actionné par un levier. Lorsqu'il est désactivé, le contrôle manuel du dispositif à aiguille est possible.
6) Un sélecteur de longueur est situé sur la partie coulissante de l'unité d'entraînement. Le sélecteur de longueur détermine la distance de passage de l'aiguille de biopsie. La longueur désirée doit être définie avant le passage de l'aiguille.
7) Pour effectuer un prélèvement de biopsie, l'endoscopiste avance l'aiguille près de l'emplacement souhaité à l'aide du contrôleur de conduite ou manuellement. Une longueur souhaitée est définie sur le sélecteur de longueur sur la partie coulissante de l'unité d'entraînement. Une aiguille est avancée dans la lésion via le bouton de contrôle de conduite. La longueur souhaitée de pénétration de l'aiguille peut être ajustée, si nécessaire, jusqu'à ce que l'aiguille arrive à l'emplacement souhaité dans la cible.
8) Une fois que l'aiguille est dans la cible et que la longueur de pénétration souhaitée est sélectionnée, plusieurs passages de l'aiguille peuvent être effectués. Lorsque l'utilisateur appuie sur le bouton d'échantillonnage, l'actionneur linéaire logé dans l'unité d'entraînement est activé. L'actionneur linéaire rapproche les connecteurs les uns des autres pendant un temps prédéfini. Ce mouvement de l'actionneur linéaire amène l'aiguille à avancer et à se rétracter à la distance prédéfinie en fonction du sélecteur de longueur.
9) Lorsqu'un jeu de passes est terminé, l'utilisateur peut repositionner manuellement l'aiguille et réajuster la longueur de pénétration souhaitée, si nécessaire. Les étapes 8 et 9 sont répétées jusqu'à ce que suffisamment d'échantillons soient collectés.
10) Documentation de la procédure d'échantillonnage - l'utilisateur enregistre chaque longueur présélectionnée et le nombre correspondant de passages effectués en appuyant sur le bouton d'échantillonnage.

### Application « pinces »

Les outils endoscopiques courants, tels que les pinces et les collets (par exemple), nécessitent l'ouverture ou la fermeture manuelle de la poignée de l'outil (voir la figure 8).

Le contrôle de la poignée de l'outil est généralement effectué par un assistant sous la supervision d'un endoscopiste et non directement par un endoscopiste. La communication, cependant, pourrait être difficile car le degré exact de fermeture / ouverture et le mouvement à effectuer pourraient être difficiles à verbaliser. Cela nécessite généralement un assistant expérimenté ou une longue courbe d'apprentissage entre un assistant et l'endoscopiste pour obtenir une communication plus efficace. Avec une bonne communication, il est encore difficile d'éviter une mauvaise communication qui pourrait entraîner des erreurs de fonctionnement, un temps de fonctionnement supplémentaire et des problèmes de sécurité. Une solution consiste à fournir un contrôle automatique lors de la fermeture et de l'ouverture de l'outil via une interface de contrôle utilisateur, afin que les endoscopistes puissent contrôler leur propre outil.
1) La solution proposée par la présente invention s'applique parfaitement à la commande d'un instrument endoscopique nécessitant une commande d'ouverture ou de fermeture et qui est fixé à un porte-outil adjacent à la poignée de l'endoscope.
2) Il se compose d'un connecteur qui s'étend à partir de la partie inférieure de l'unité d'entraînement et d'un porte-outil qui se connecte au connecteur.
3) Le porte-outil est constitué d'un actionneur linéaire en deux parties: une partie coulissante et une partie non coulissante. Au moins une partie de l'outil endoscopique est montée sur le porte-outil.
4) Les outils endoscopiques ouverts et fermés consistent généralement en une partie coulissante et non coulissante (non mobile). Une partie coulissante du dispositif est montée sur la partie coulissante de l'outil; tandis que la partie non glissante de l'appareil est montée sur la partie non glissante de l'outil.
5) L'ouverture ou la fermeture de l'outil est contrôlée par la position / le mouvement de la pièce coulissante, qui peut être contrôlé par l'utilisateur via une interface de commande utilisateur (similaire à la commande d'origine) ou des pédales.

### Modulation de la vitesse de déplacement de l'instrument endoscopique

Certaines procédures endoscopiques nécessitent des contrôles de vitesse différents, notamment une vitesse lente pour un contrôle précis des mouvements; et un avancement rapide qui permet la perforation à travers la membrane d'une structure. L invention permet selon une variante de prévoir deux boutons de contrôle de vitesse différents sur l'interface de contrôle utilisateur, qui permettent un contrôle plus précieux de la vitesse de l'appareil, ou encore un capteur indexé permettant de sélectionner la vitesse adéquate à l'aide du bouton de commande (114).

### Configuration du module

La configuration du talon (112) du module d'actionnement peut prendre différentes formes, en fonction de la nature du capteur qu'il supporte. A titre d'exemples non limitatifs, six configurations particulières sont présentées ci-après.

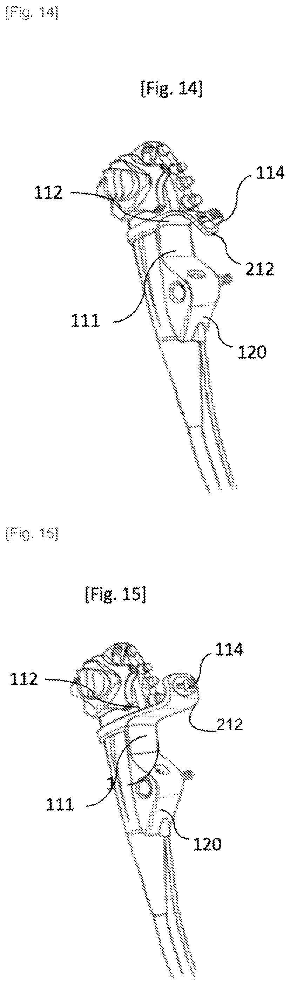

La figure 14 est une vue en perspective d'une première configuration d'une platine selon l'invention. Le talon (112) s'étend dans une direction sensiblement perpendiculaire à la surface d'appui (111), puis présente une zone recourbée, dans la direction du bloc d'entraînement motorisé (120) pour former une surface (212) sensiblement parallèle (ou légèrement inclinée avec un angle de l'ordre de 30°) supportant un capteur muni d'un bouton qui peut être actionné latéralement, de droite à gauche, et appuyé, par exemple pour commander l'avancement ou le recul d'un outil et son blocage.

La figure 15 est une vue en perspective d'une deuxième configuration d'une platine selon l'invention similaire à la configuration précédente, sauf que le recourbement est dans la direction opposée au bloc motorisé.

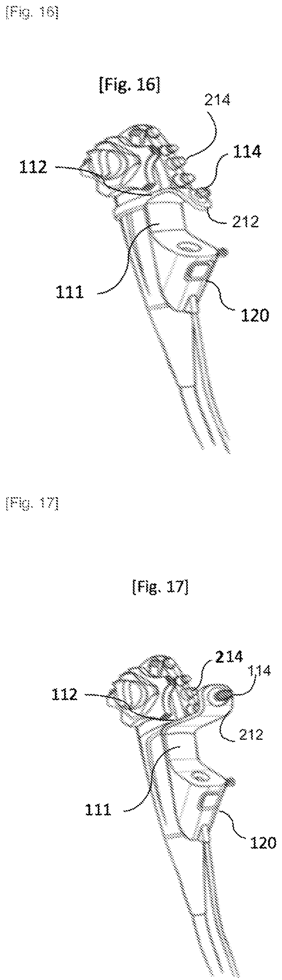

La figure 16 est une vue en perspective d'une troisième configuration d'une platine selon l'invention où le recourbement est dans le même sens que dans la première configuration, la surface (212) supportant une molette actionnables dans différentes direction, de type « joystick », avec un actionnement possible en poussée.

La figure 17 est une vue en perspective d'une quatrième configuration d'une platine selon l'invention où un deuxième actionneur (214) est monté sur une platine (212) recourbée reliée au bloc moteur (120).

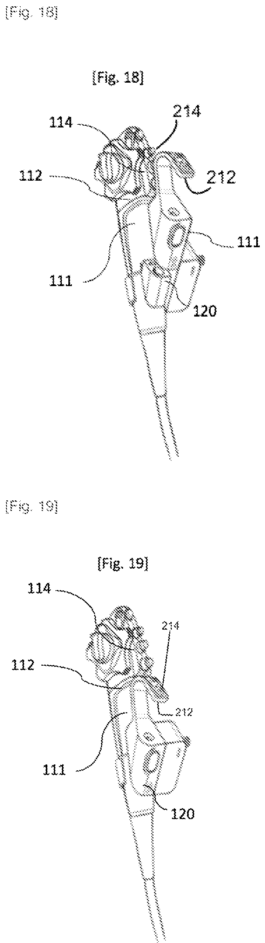

La figure 18 est une vue en perspective d'une cinquième configuration d'une platine selon l'invention avec une deuxième platine recourbée (212) supportant plusieurs capteurs ou bouton (214) superposé au premier capteur (114), les deux capteurs (114) et (214) étant dans des plans décalés.

La figure 19 est une vue en perspective d'une sixième configuration d'une platine selon l'invention avec une deuxième platine recourbée (212) supportant plusieurs capteurs ou bouton (214) décalé longitudinalement par rapport au premier capteur (114), les deux capteurs (114) et (214) étant dans des plans décalés.

## Revendications

1. - Module d'actionnement motorisé d'un instrument endoscopique **caractérisé en ce qu'**il est constitué par une platine (100) apte à être fixée sur la zone de préhension cubito-palmaire d'une poignée d'un endoscope, ladite platine (100) présentant une surface d'appui cubito-palmaire (111) prolongée par un talon (112) s'étendant dans une direction formant un angle de 90° ± 25° par rapport au plan de ladite surface d'appui cubito-palmaire (111) , ledit talon (112) comportant un capteur électro-mécanique (114) délivrant un signal de commande du déplacement d'un instrument endoscopique.

2. - Module d'actionnement motorisé d'un instrument endoscopique selon la revendication 1 **caractérisé en ce que** ladite surface d'appui cubito-palmaire médiane (111) est prolongée du coté opposée par un bloc d'entraînement motorisé (120), comportant un mécanisme motorisé d'un élément filiforme de liaison avec ledit instrument dont l'extrémité inférieure débouche dans la tige flexible (1) d'un endoscope pour assurer une liaison avec ledit instrument, ladite platine présentant en outre des moyens de liaison avec la poignée d'un endoscope flexible.

3. - Module d'actionnement motorisé d'un instrument endoscopique selon la revendication précédente **caractérisé en ce que** lesdits moyens de liaison avec la poignée d'un endoscope flexible sont constitués par un embout (271) apte à être insérré dans le canal opérateur de ladite tige flexible (1) de l'endoscope.

4. - Module d'actionnement motorisé d'un instrument endoscopique selon la revendication 2 **caractérisé en ce que** ledit bloc d'entraînement motorisé (120) comporte un moteur (190) placé en-dessous de l'embout (271).

5. - Module d'actionnement motorisé d'un instrument endoscopique selon la revendication 1 **caractérisé en ce que** ledit talon (112) est formé par une protubérance (112) d'épaisseur inférieure à 3 millimètres présentant à son extrémité un capteur (114) dont la surface d'actionnement est définie par une génératrice formant un angle de 90° ± 20° avec l'axe longitudinal de ladite poignée.

6. - Module d'actionnement motorisé d'un instrument endoscopique selon la revendication 1 **caractérisé en ce qu'**il comprend en outre dans la partie inférieure de ladite zone de préhension cubito-palmaire, un bloc d'entraînement (120), comportant un mécanisme motorisé d'un élément filiforme (160) de liaison avec ledit instrument dont l'extrémité inférieure débouche dans ladite tige flexible (1) de l'endoscope pour assurer une liaison avec ledit instrument

7. - Module d'actionnement motorisé d'un instrument endoscopique selon la revendication précédente **caractérisé en ce que** ledit bloc d'entrainement présente un bouton d'actionnement (124) latéral commandant l'arrêt d'urgence du déplacement dudit instrument.

8. - Endoscope souple comportant une poignée (1) présentant des boutons de commande (20, 30) de l'aspiration et du lavage située au-dessus d'une zone de préhension cubito-palmaire des doigts de l'utilisateur, ladite poignée (1) étant prolongée par une tige flexible (2) présentant un canal opérateur pour le passage d'un instrument dont le déplacement est commandé par une interface électrique **caractérisé en ce que** ladite interface (100) présente une surface d'appui cubito-palmaire (111) prolongée par un talon (112) s'étendant dans une direction formant un angle de 90° ± 25° par rapport au plan de ladite surface d'appui cubito-palmaire (111) , ledit talon comportant un capteur électro-mécanique (114) délivrant un signal de commande du déplacement d'un instrument endoscopie, l'autre extrémité de l'interface (100) étant formée par un bloc d'entraînement (120).

9. - Endoscope souple selon la revendication 8 **caractérisé en ce que** ledit talon (112) est formé par une protubérance (112) d'épaisseur inférieure à 3 millimètres présentant à son extrémité un capteur (114) dont la surface d'actionnement est définie par une génératrice formant un angle de 90° ± 20° avec l'axe longitudinal de ladite poignée.

10. - Endoscope souple selon la revendication précédente **caractérisé en ce que** ledit capteur (114) est un capteur rotatif actionné par une molette dont l'axe forme un angle compris entre 0° et ±70° par rapport à l'axe longitudinal parallèle à l'axe longitudinal de ladite poignée.

11. - Endoscope souple selon la revendication 8 **caractérisé en ce qu'**il comprend en outre dans la partie inférieure de ladite zone de préhension cubito-palmaire, un bloc d'entraînement (120), comportant un mécanisme motorisé d'un élément filiforme (160) de liaison avec ledit instrument dont l'extrémité inférieure débouche dans ladite tige flexible (1) de l'endoscope pour assurer une liaison avec ledit instrument.

12. - Endoscope souple selon la revendication 8 **caractérisé en ce que** ledit bloc d'entrainement (120) forme une protubérance par rapport à la surface de la partie inférieure de la zone de préhension cubito-palmaire, à l'opposé, par rapport à ladite zone de préhension cubito-palmaire, audit prolongement consituant la commande du déplacement dudit instrument.

13. - Endoscope souple selon la revendication précédente **caractérisé en ce que** ledit bloc d'entrainement présente un bouton d'actionnement (124) latéral commandant l'arrêt d'urgence du déplacement dudit instrument.

14. - Endoscope souple selon la revendication 8 **caractérisé en ce que** ladite interface de commande (100) comporte en outre un sélecteur de longueur pour le déplacement de l'instrument d'une longeur prédéterminée.

15. - Endoscope souple selon la revendication 8 **caractérisé en ce que** ladite interface de commande comporte en outre un moyen d'accouplement (131) d'un périphérique dudit instrument.

16. - Endoscope souple selon la revendication 8 **caractérisé en ce qu'**il comporte un module d'actionnement (100) motorisé regroupant sous forme d'un sous-ensemble détachable de la poignée ladite interface et ledit bloc d'entraînement.

17. - Endoscope souple selon la revendication 8 **caractérisé en ce que** ledit capteur (114) présentant une surface d'interaction opposée à ladite zone de préhension cubito-palmaire inférieure à 50 mm².

## Patentansprüche

1. Motorisiertes Betätigungsmodul eines endoskopischen Instruments, **dadurch gekennzeichnet, dass** es aus einer Platte (100) besteht, die geeignet ist, an dem ulnar-palmaren Griffbereich eines Griffs eines Endoskops befestigt zu werden, wobei die Platte (100) eine ulnar-palmare Stützfläche (111) aufweist, die durch eine Ferse (112) verlängert ist, die sich in einer Richtung erstreckt, die einen Winkel von 90° ± 25° zur Ebene der ulnar-palmaren Stützfläche (111) bildet, wobei die Ferse (112) einen elektromechanischen Sensor (114) enthält, der ein Signal zur Steuerung der Bewegung eines endoskopischen Instruments liefert.

2. Motorisiertes Betätigungsmodul eines endoskopischen Instruments nach Anspruch 1, **dadurch gekennzeichnet, dass** die mittlere ulnar-palmare Stützfläche (111) auf der gegenüberliegenden Seite durch einen motorisierten Antriebsblock (120) verlängert ist, enthaltend einen motorisierten Mechanismus aus einem drahtförmigen Verbindungselement mit dem Instrument, dessen unteres Ende in den flexiblen Schaft (1) eines Endoskops mündet, um eine Verbindung mit dem Instrument sicherzustellen, wobei die Platte weiter Mittel zur Verbindung mit dem Griff eines flexiblen Endoskops aufweist.

3. Motorisiertes Betätigungsmodul eines endoskopischen Instruments nach dem vorstehenden Anspruch, **dadurch gekennzeichnet, dass** die Verbindungsmittel mit dem Griff eines flexiblen Endoskops aus einem Ansatzstück (271) bestehen, das in den Arbeitskanal des flexiblen Schafts (1) des Endoskops eingeführt werden kann.

4. Motorisiertes Betätigungsmodul eines endoskopischen Instruments nach Anspruch 2, **dadurch gekennzeichnet, dass** der motorisierte Antriebsblock (120) einen Motor (190) enthält, der unterhalb des Ansatzstücks (271) angeordnet ist.

5. Motorisiertes Betätigungsmodul eines endoskopischen Instruments nach Anspruch 1, **dadurch gekennzeichnet, dass** die Ferse (112) durch einen Vorsprung (112) mit einer Dicke von weniger als 3 Millimetern gebildet wird, der an seinem Ende einen Sensor (114) aufweist, dessen Betätigungsfläche durch eine Erzeugende definiert ist, die einen Winkel von 90° ± 20° mit der Längsachse des Griffs bildet.

6. Motorisiertes Betätigungsmodul eines endoskopischen Instruments nach Anspruch 1, **dadurch gekennzeichnet, dass** es im unteren Teil des ulnar-palmaren Griffbereichs weiter einen Antriebsblock (120) umfasst, der einen motorisierten Mechanismus eines fadenförmigen Verbindungselements (160) mit dem Instrument enthält, dessen unteres Ende in den flexiblen Schaft (1) des Endoskops mündet, um eine Verbindung mit dem Instrument sicherzustellen

7. Motorisiertes Betätigungsmodul eines endoskopischen Instruments nach dem vorstehenden Anspruch, **dadurch gekennzeichnet, dass** der Antriebsblock einen seitlichen Betätigungsknopf (124) aufweist, der den Notstopp der Bewegung des Instruments steuert.

8. Flexibles Endoskop, das einen Griff (1) enthält, der Bedienknöpfe (20, 30) für die Absaugung und Spülung aufweist, die sich oberhalb eines ulnar-palmaren Griffbereichs für die Finger des Benutzers befinden, wobei der Griff (1) durch einen flexiblen Schaft (2) verlängert ist, der einen Arbeitskanal für den Durchgang eines Instruments aufweist, dessen Bewegung durch eine elektrische Schnittstelle gesteuert wird, **dadurch gekennzeichnet, dass** die Schnittstelle (100) eine ulnar-palmare Stützfläche (111) aufweist, die durch eine Ferse (112) verlängert wird, der sich in einer Richtung erstreckt, die einen Winkel von 90° ± 25° in Bezug auf die Ebene der ulnar-palmaren Stützfläche (111) bildet, wobei die Ferse einen elektromechanischen Sensor (114) enthält, der ein Signal zur Steuerung der Bewegung eines endoskopischen Instruments liefert, wobei das andere Ende der Schnittstelle (100) durch einen Antriebsblock (120) gebildet wird.

9. Flexibles Endoskop nach Anspruch 8, **dadurch gekennzeichnet, dass** die Ferse (112) durch einen Vorsprung (112) mit einer Dicke von weniger als 3 Millimetern gebildet wird, der an seinem Ende einen Sensor (114) aufweist, dessen Betätigungsfläche durch eine Mantellinie definiert ist, die einen Winkel von 90° ± 20° mit der Längsachse des Griffs bildet.

10. Flexibles Endoskop nach dem vorstehenden Anspruch, **dadurch gekennzeichnet, dass** der Sensor (114) ein Drehsensor ist, der durch ein Rädchen betätigt wird, dessen Achse einen Winkel zwischen 0° und ±70° in Bezug auf die Längsachse parallel zur Längsachse des Griffs bildet.

11. Flexibles Endoskop nach Anspruch 8, **dadurch gekennzeichnet, dass** es im unteren Teil des ulnar-palmaren Griffbereichs weiter einen Antriebsblock (120) umfasst, der einen motorisierten Mechanismus eines fadenförmigen Verbindungselements (160) mit dem Instrument enthält, dessen unteres Ende in den flexiblen Schaft (1) des Endoskops mündet, um eine Verbindung mit dem Instrument sicherzustellen.

12. Flexibles Endoskop nach Anspruch 8, **dadurch gekennzeichnet, dass** der Antriebsblock (120) einen Vorsprung gegenüber der Oberfläche des unteren Teils des ulnar-palmaren Griffbereichs bildet, der dem ulnar-palmaren Griffbereich gegenüberliegt, wobei diese Verlängerung die Steuerung der Bewegung des Instruments bildet.

13. Flexibles Endoskop nach dem vorstehenden Anspruch, **dadurch gekennzeichnet, dass** der Antriebsblock einen seitlichen Betätigungsknopf (124) aufweist, der die Notabschaltung der Bewegung des Instruments steuert.

14. Flexibles Endoskop nach Anspruch 8, **dadurch gekennzeichnet, dass** die Steuerschnittstelle (100) weiter einen Längenselektor zum Verschieben des Instruments um eine vorbestimmte Länge enthält.

15. Flexibles Endoskop nach Anspruch 8, **dadurch gekennzeichnet, dass** die Steuerschnittstelle weiter eine Kopplungseinrichtung (131) für ein Peripheriegerät des Instruments enthält.

16. Flexibles Endoskop nach Anspruch 8, **dadurch gekennzeichnet, dass** es ein motorisiertes Betätigungsmodul (100) enthält, das die Schnittstelle und den Antriebsblock in Form einer vom Griff abnehmbaren Unterbaugruppe zusammenfasst.

17. Flexibles Endoskop nach Anspruch 8, **dadurch gekennzeichnet, dass** der Sensor (114) eine Interaktionsfläche gegenüber dem ulnar-palmaren Griffbereich von weniger als 50 mm² aufweist.

## Claims

1. A motorized actuation module of an endoscopic instrument, **characterized in that** it consists of a plate (100), adapted to be fixed on the ulnar-palmar grasping zone of a handle of an endoscope, wherein said plate (100) has an ulnar-palmar support surface (111) extended by a heel (112), extending in a direction forming an angle of 90° ± 25° with respect to the plane of said ulnar-palmar support surface (111), said heel (112) comprising an electro-mechanical sensor (114) delivering a control signal for the movement of an endoscopic instrument.

2. The motorized actuation module of an endoscopic instrument according to claim 1, **characterized in that** said median ulnar-palmar support surface (111) is extended on the opposite side by a motorized drive block (120), comprising a motorized mechanism of a wire element for connecting with said instrument, the lower end of which opens into the flexible rod (1) of an endoscope to ensure a connection with said instrument, said plate further having means for connecting with the handle of a flexible endoscope.

3. The motorized actuation module of an endoscopic instrument according to the preceding claim, **characterized in that** said means of connection with the handle of a flexible endoscope are constituted by a tip (271), suitable for being inserted into the working channel of said flexible rod (1) of the endoscope.

4. The motorized actuation module of an endoscopic instrument according to claim 2, **characterized in that** said motorized drive block (120) comprises a motor (190) placed below the tip (271).

5. The motorized actuation module of an endoscopic instrument according to claim 1, **characterized in that** said heel (112) is formed by a protrusion (112) of a thickness less than 3 millimeters having at its end a sensor (114) whose actuation surface is defined by a generatrix, forming an angle of 90° ± 20° with the longitudinal axis of said handle.

6. The motorized actuation module of an endoscopic instrument according to claim 1, **characterized in that** it further comprises, in the lower part of said ulnar-palmar gripping zone, a drive block (120) comprising a motorized mechanism for a filament element (160) for connection with said instrument, the lower end of which opens into said flexible rod (1) of the endoscope to ensure a connection with said instrument.

7. The motorized actuation module of an endoscopic instrument according to the preceding claim, **characterized in that** said drive block has a lateral actuation button (124) controlling the emergency stop of the movement of said instrument.

8. A flexible endoscope comprising a handle (1) having control buttons (20, 30) for suction and washing, located above an ulnar-palmar grasping zone for the user's fingers, said handle (1) being extended by a flexible rod (2) having an operating channel for the passage of an instrument whose movement is controlled by an electrical interface, **characterized in that** said interface (100) has an ulnar-palmar support surface (111), extended by a heel (112), extending in a direction forming an angle of 90° ± 25° with respect to the plane of said ulnar-palmar support surface (111), said heel comprising an electro-mechanical sensor (114), delivering a control signal for the movement of an endoscopic instrument, the other end of the interface (100) being formed by a drive block (120).

9. The flexible endoscope according to claim 8, **characterized in that** said heel (112) is formed by a protrusion (112) of thickness less than 3 millimeters having at its end a sensor (114) whose actuation surface is defined by a generatrix, forming an angle of 90° ± 20° with the longitudinal axis of said handle.

10. The flexible endoscope according to the preceding claim, **characterized in that** said sensor (114) is a rotary sensor, actuated by a wheel whose axis forms an angle between 0° and ±70° with respect to the longitudinal axis parallel to the longitudinal axis of said handle.

11. The flexible endoscope according to claim 8, **characterized in that** it further comprises, in the lower part of said ulnar-palmar gripping zone, a drive block (120), comprising a motorized mechanism of a filiform element (160) for connection with said instrument, the lower end of which opens into said flexible rod (1) of the endoscope to ensure a connection with said instrument.

12. The flexible endoscope according to claim 8, **characterized in that** said drive block (120) forms a protrusion with respect to the surface of the lower part of the ulnar-palmar gripping zone, opposite, with respect to said ulnar-palmar gripping zone, said extension constituting the control of the movement of said instrument.

13. The flexible endoscope according to the preceding claim, **characterized in that** said drive block has a lateral actuation button (124) for controlling the emergency stop of the movement of said instrument.

14. The flexible endoscope according to claim 8, **characterized in that** said control interface (100) further comprises a length selector for moving the instrument by a predetermined length.

15. The flexible endoscope according to claim 8, **characterized in that** said control interface further comprises a coupling means (131) for a peripheral of said instrument.

16. The flexible endoscope according to claim 8, **characterized in that** it comprises a motorized actuation module (100), grouping together said interface and said drive block in the form of a detachable subassembly of the handle.

17. The flexible endoscope according to claim 8, **characterized in that** said sensor (114) has an interaction surface opposite said ulnar-palmar gripping zone of less than 50 mm².
